# EUROPÄISCHE PATENTSCHRIFT

(11) **EP 3 451 979 B1**
(45) Veröffentlichungstag und Bekanntmachung des Hinweises auf die Patenterteilung: **13.05.2020**
(21) Anmeldenummer: 17720454.2
(22) Anmeldetag: 26.04.2017
(51) Int. Cl.: A61F 5/01

(54) **GELENK FÜR EINE ORTHOPÄDIETECHNISCHE EINRICHTUNG**
JOINT FOR AN ORTHOPEDIC DEVICE
ARTICULATION POUR UN DISPOSITIF ORTHOPÉDIQUE

(30) Priorität: 02.05.2016 DE 102016108107
(43) Veröffentlichungstag der Anmeldung: 13.03.2019
(73) Patentinhaber: Ottobock SE & Co. KGaA, 37115 Duderstadt (DE)
(72) Erfinder: LÜRSSEN, Marcus, 37073 Göttingen (DE)
(74) Vertreter: Gramm, Lins & Partner Patent- und Rechtsanwälte PartGmbB
(86) Internationale Anmeldenummer: PCT/EP2017/059917
(87) Internationale Veröffentlichungsnummer: WO 2017/191006

(56) Entgegenhaltungen:
- WO-A1-99/11206
- WO-A2-2006/048012
- DE-A1-102013 011 382
- DE-U1- 29 813 360
- FR-A- 506 846
- FR-A1- 2 441 382
- US-A- 5 460 599

## Beschreibung

Die Erfindung betrifft ein Gelenk für eine orthopädietechnische Einrichtung, das einen ersten Gelenkarm mit einer Ausnehmung, einen zweiten Gelenkarm, der in die Ausnehmung hineinragt und darin mit dem ersten Gelenkarm um eine Schwenkachse schwenkbar verbunden ist und wenigstens ein Abstandselement aufweist, das innerhalb der Ausnehmung zwischen dem ersten Gelenkarm und dem zweiten Gelenkarm angeordnet ist und durch das die Schwenkachse verläuft, wobei das wenigstens eine Abstandselement von außen zugänglich ist, insbesondere aus der Ausnehmung herausragt.

Derartige Gelenke werden in einer Vielzahl orthopädietechnischer Einrichtungen, beispielsweise Knöchelorthesen, verwendet. Der erste Gelenkarm verfügt über eine Ausnehmung, in die der zweite Gelenkarm hineinragt. Dadurch können die seitlichen Begrenzungswände der Ausnehmung als Anschlagselemente verwendet werden, durch die eine Schwenkbewegung der beiden Gelenkarme relativ zueinander begrenzt werden kann. Selbstverständlich ist es jedoch auch möglich, zusätzliche Anschlagselemente und/oder Dämpfungselemente zu verwenden. Zwischen dem ersten Gelenkarm und dem zweiten Gelenkarm ist innerhalb dieser Ausnehmung in der Regel wenigstens ein Abstandselement, beispielsweise eine Unterlegscheibe, angeordnet.

Oftmals verfügen beide Gelenkarme über eine Bohrung, die zur Montage des Gelenkes in Überdeckung gebracht werden müssen. In dieser Position lässt sich ein Verbindungselement, beispielsweise ein Bolzen, ein Stift oder eine Schraube, durch die Bohrungen beider Gelenkarme hindurchführen. Dadurch wird die schwenkbare Verbindung der beiden Gelenkarme miteinander erreicht. Um Fertigungstoleranzen auszugleichen und/oder zu verhindern, dass die beiden Gelenkarme vollflächig aneinander anliegen, ist innerhalb der Ausnehmung wenigstens ein Abstandselement, beispielsweise in Form einer Unterlegscheibe angeordnet. Zum Montieren des Gelenkes muss diese Unterlegscheibe innerhalb der Ausnehmung so positioniert werden, dass das Verbindungselement nicht nur durch die Bohrungen der beiden Gelenkarme, sondern auch durch eine dafür vorgesehene Bohrung in der Unterlegscheibe hindurchgeführt werden kann. Dazu muss die Unterlegscheibe folglich relativ zum ersten Gelenkarm und relativ zum zweiten Gelenkarm bewegt werden. Dies muss jedoch innerhalb der Ausnehmung geschehen, was aufgrund der sehr beengten Platzverhältnisse und der schlechten Zugänglichkeit nur schwer und mit großem Aufwand möglich ist.

Eine solche Ausgestaltung, bei der die Unterlegscheibe einen ringförmigen Vorsprung aufweist, mit dem sie in die die Bohrung eines Gelenkarmes einrasten kann, ist aus der WO 2006/048012 bekannt. Aus der US 5,460,599 sind Unterlegscheiben bekannt, die aus der Ausnehmung herausragen und als Anschlag für die Verschwenkung der beiden Gelenkarme relativ zueinander dienen.

Der Erfindung liegt daher die Aufgabe zugrunde, ein gattungsgemäßes Gelenk gemäß dem Oberbegriff des Anspruchs 1 so weiter zu entwickeln, dass die Montage einfacher und schneller möglich ist.

Die Erfindung löst die gestellte Aufgabe durch ein Gelenk gemäß dem Oberbegriff des Anspruchs 1, das sich dadurch auszeichnet, dass wenigstens eines der Abstandselemente einen Anschlag aufweist, der verhindert, dass das Abstandselement vollständig in der Ausnehmung zwischen dem ersten Gelenkarm und dem zweiten Gelenkarm aufgenommen werden kann, und dass der Anschlag so ausgebildet ist, dass er an einem Rand der Ausnehmung anliegt. Auf diese Weise ist ein Teil des Abstandselementes nicht nur sichtbar, sondern kann manuell oder mit einem Werkzeug gegriffen und bewegt und in einer gewünschten Position fixiert werden. Auf diese Weise ist die Positionierung des Abstandselementes deutlich vereinfacht und der Herstellungsprozess des Gelenkes kann beschleunigt werden.

Die Ausnehmung kann einen Rand aufweisen, der beispielsweise eine konvexe Form aufweist, also beispielsweise in Richtung auf den zweiten Gelenkarm gewölbt ausgebildet ist. Dies ist jedoch nicht zwingend notwendig. So ist es auch denkbar, dass in dieser Kontur des Randes beispielsweise eine oder mehrere Buchten oder Taschen ausgebildet sind, die einen konkaven Anteil dieses Randes bilden. In diesem Fall ist es möglich, das Abstandselement so auszubilden, dass es zwar von außen zugänglich ist, jedoch nicht über eine konvexe Vervollständigung der Kontur des Randes der Ausnehmung hinausragt. Trotzdem ist diese Ausgestaltung selbstverständlich erfindungsgemäß, da das Abstandselement von außen zugänglich ist.

Vorzugsweise verfügt das Gelenk über zwei Abstandselemente, die auf unterschiedlichen Seiten des zweiten Gelenkarmes in der Ausnehmung angeordnet sind. Dabei sind vorzugsweise beide Abstandselemente so ausgebildet, dass sie aus der Ausnehmung des ersten Gelenkarmes herausragen, um eine Positionierung und auch eine Fixierung während der Montage zu gewährleisten, so dass ein gegebenenfalls einzuführendes Verbindungselement leicht, einfach und sicher eingeführt werden kann.

Erfindungsgemäß verfügt wenigstens eines der Abstands-elemente über einen Anschlag, der verhindert, dass das Abstandselement vollständig in der Ausnehmung zwischen dem ersten Gelenkarm und dem zweiten Gelenkarm aufgenommen werden kann. Ein derartiger Anschlag kann beispielsweise in einem winkelig angeordneten Endstück oder einer Verdickung bestehen. Vorzugsweise verfügen alle verwendeten Abstandselemente über einen derartigen Anschlag.

Vorzugsweise ist das Abstandselement verdrehsicher zu dem ersten Gelenkarm gelagert. Dies kann besonders einfach durch den Anschlag geschehen, der an dem ersten Gelenkarm, beispielsweise am Rand der Ausnehmung, anliegt und auf diese Weise ein Verdrehen des Abstandselementes relativ zu diesem Gelenkarm verhindert. Alternativ dazu, technisch jedoch aufwendiger zu realisieren, ist es selbstverständlich auch möglich, das wenigstens eine Abstandselement verdrehsicher an dem zweiten Gelenkarm zu lagern.

Auf diese Weise wird verhindert, dass bei der Montage versehentlich eines der Abstandselemente vollständig zwischen den beiden Gelenkarmen in der Ausnehmung aufgenommen wird und sich so einer weiteren Positionierung oder Fixierung entzieht.

Vorteilhafterweise ist der Anschlag so ausgebildet, dass er einer Kontur des Randes folgt und insbesondere formschlüssig an der Kontur anliegt.

Vorzugsweise verfügt das Gelenk über ein Verbindungselement, durch das der erste Gelenkarm mit dem zweiten Gelenkarm verbunden ist. Vorteilhafterweise verfügt auch das wenigstens eine Abstandselement über eine Bohrung, durch die das Verbindungselement hindurch verläuft, wenn das Gelenk montiert ist. Durch ein derartiges Verbindungsgelenk, das beispielsweise als Schraube, Hülse, Bolzen oder Stift ausgebildet sein kann, wird einerseits die schwenkbare Verbindung der beiden Gelenkarme miteinander und gleichzeitig eine Positionierung der verwendeten Bauteile zueinander gewährleistet.

Erfindungsgemäß ist der Anschlag an wenigstens einem der Abstandselemente so ausgebildet, dass er an einem Rand der Ausnehmung anliegt wenn das Gelenk montiert ist. Diese Ausgestaltung hat mehrere Vorteile. Einerseits wird gewährleistet, dass das Abstandselement gerade so groß ist, wie es benötigt wird, um nicht vollständig in der Ausnehmung aufgenommen zu werden. Dies bedeutet, dass das Abstandselement möglichst klein und damit mit möglichst wenig Materialaufwand ausgebildet sein kann. Gleichzeitig wird durch den an dem Rand der Ausnehmung anliegenden Anschlag erreicht, dass eine Positionierung des wenigstens einen Abstandselementes bei der Montage besonders einfach möglich ist. Es muss lediglich das wenigstens eine Abstandselement soweit zwischen den beiden Gelenkarmen in die Ausnehmung eingeschoben werden, bis ein Anschlag am Rand der Ausnehmung anliegt. Dadurch wird zumindest eine Vorpositionierung ermöglicht, so dass eine spätere feine Einstellung der Positionierung nur noch eine leichte Verschiebung enthalten muss oder ganz entfallen kann.

Es hat sich als vorteilhaft herausgestellt, wenn das wenigstens eine Abstandselement aus einem Kunststoff besteht. Dadurch können einerseits Klappergeräusche innerhalb des Gelenkes vermieden werden, die ansonsten beispielsweise bei metallischen Gelenkarmen auftreten können. Gleichzeitig wird ein Reibungswiderstand beim Verschwenken des Gelenkes verringert und das Gelenk wird insbesondere bei Verwendung in einer orthopädietechnischen Einrichtung als leichtgängig empfunden.

Die Erfindung löst die gestellte Aufgabe zudem durch eine orthopädietechnische Einrichtung mit wenigstens einem hier beschriebenen Gelenk. Vorteilhafterweise verfügt die orthopädietechnische Einrichtung über zwei derartige Gelenke, die vorzugsweise auf unterschiedlichen Seiten eines Körperteils, insbesondere eines Gelenkes, angeordnet werden können. Bevorzugt handelt es sich bei der orthopädietechnischen Einrichtung um ein Schienensystem für eine Orthese oder eine Knöchelorthese. Selbstverständlich kann das Gelenk jedoch auch bei anderen orthopädietechnischen Einrichtungen Verwendung finden.

Das Abstandselement für ein erfindungsgemäßes Gelenk sollte möglichst gute Gleiteigenschaften, also einen möglichst geringen Reibungskoeffizienten aufweisen und zudem möglichst stabil, also verschleißfest, sein. Dazu kann es beispielsweise beschichtet oder aus einem sehr gleitfähigen Material hergestellt sein. Insbesondere bei der Verwendung einer Beschichtung können die Gleiteigenschaften sowie beispielsweise die Abriebfestigkeit und die Abrasionsbeständigkeit gleichzeitig optimiert werden.

Die Gelenke für orthopädietechnische Einrichtungen gemäß der vorliegenden Erfindung können für Orthesen und Prothesen für unterschiedlichste Körperteile verwendet werden. Sie können als Knie-, Knöchel-, Hüft-, Ellenbogen- oder Handgelenke verwendet werden oder auch an sonstigen Positionen von Orthesen und/oder Prothesen verwendet werden.

Mit Hilfe der beigefügten Zeichnungen wird nachfolgend ein Ausführungsbeispiel der vorliegenden Erfindung näher erläutert. Es zeigt:
- Figur 1: - die schematische Ansicht eines Gelenkes gemäß einem ersten Ausführungsbeispiel der vorliegenden Erfindung,
- Figur 2: - das Gelenk aus Figur 1 in einer Schnittdarstellung,
- Figur 3: - ein vergrößerter Ausschnitt des Gelenkes aus den Figuren 1 und 2 in einer schematischen dreidimensionalen Ansicht,
- Figur 4: - das Gelenk aus den Figuren 1 bis 3 in teilweise demontieren Zustand und
- Figur 5: - ein Abstandselement gemäß einem Ausführungsbeispiel der vorliegenden Erfindung in drei unterschiedlichen Ansichten.

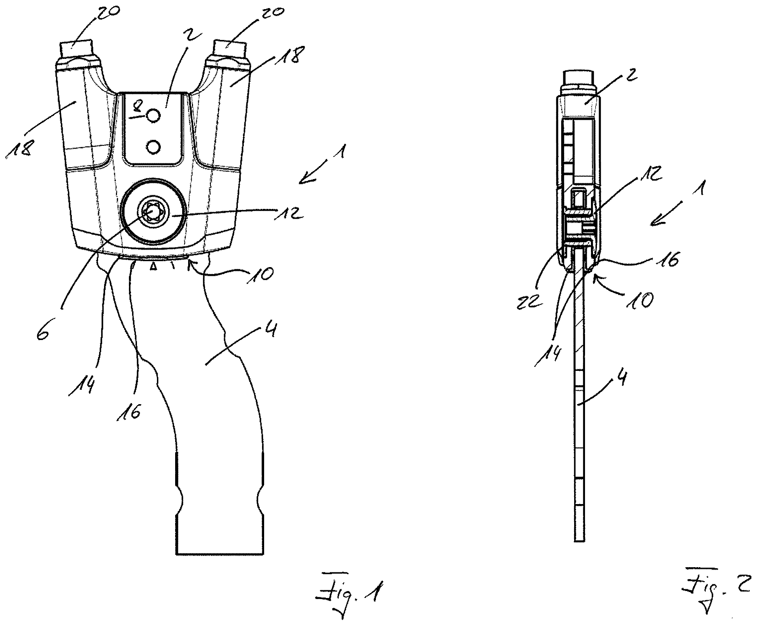

Figur 1 zeigt ein Gelenk 1 gemäß einem ersten Ausführungsbeispiel der vorliegenden Erfindung. Es verfügt über einen ersten Gelenkarm 2 und einen zweiten Gelenkarm 4, die miteinander um eine Schwenkachse 6 schwenkbar verbunden sind. Der erste Gelenkarm 2 verfügt über eine Aufnahmeeinrichtung 8 an der ein weiteres Bauteil, beispielsweise eine Schiene für eine orthopädietechnische Einrichtung, angeordnet werden kann.

Der erste Gelenkarm 2 verfügt zudem über eine Ausnehmung 10, in die der zweite Gelenkarm 4 hineinragt. Über ein Verbindungselement 12, das durch die beiden Gelenkarme 2, 4 hindurchgeführt ist, sind die Gelenkarme 2 und 4 miteinander verbunden.

Innerhalb der Ausnehmung 10 erkennt man in Figur 1 zwischen dem ersten Gelenkarm 2, also der oberen Wandung der Ausnehmung 10, und dem zweiten Gelenkarm 4 ein Abstandselement 14. Dieses ragt mit einem Anschlag 16 aus der Ausnehmung 10 heraus.

Am ersten Gelenkarm 2 sind zudem zwei Aufnahmehülsen 18 angeordnet, in die ein beispielsweise elastisches Anschlagselement einsteckbar ist, das den Schwenkwinkel, um den der zweite Gelenkarm 4 relativ zum ersten Gelenkarm 2 verschwenkbar ist, begrenzt. Über Einstelleinrichtungen 20 lässt sich die Härte eines solchen Anschlags einstellen.

Figur 2 zeigt das Gelenk 1 in einer Schnittdarstellung. Man erkennt den ersten Gelenkarm 2, den zweiten Gelenkarm 4 sowie das Verbindungselement 12, das durch die beiden Gelenkarme 2, 4 hindurchgeführt ist. Von der in Figur 2 linken Seite des ersten Gelenkarmes 2 ist ein Verbindungsgegenelement 22 in die dafür vorhandene Bohrungen eingeführt und mit dem Verbindungselement 12 verschraubt. Dadurch wird eine Befestigung der beiden Gelenkarme 2, 4 aneinander gewährleistet. Auf beiden Seiten des zweiten Gelenkarmes 4 innerhalb der Ausnehmung 10 befindet sich jeweils ein Abstandselement 14, das mit dem jeweiligen Anschlag 16 an einem Rand der Ausnehmung 10 anliegt.

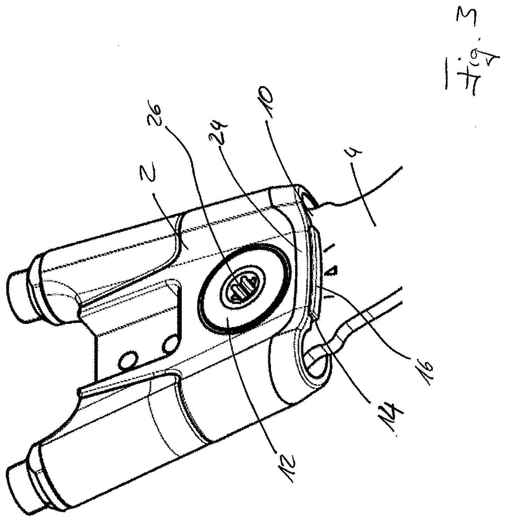

Figur 3 zeigt eine vergrößerte Darstellung in einer schematischen dreidimensionalen Ansicht. Hier ist die Ausnehmung 10, in die der zweite Gelenkarm 4 eingeschoben ist, gut zu erkennen. Man erkennt zudem den Anschlag 16 des Verbindungselementes 12, das ebenfalls in die Ausnehmung 10 eingeschoben ist. Es befindet sich zwischen dem ersten Gelenkarm 2 und dem zweiten Gelenkarm 4. Dies bedeutet, dass sich auf der einen Seite des Verbindungselementes 12 der zweite Gelenkarm 4 und auf der anderen Seite zumindest ein Teil des ersten Gelenkarms 2, vorliegend nämlich eine obere Wandung 24 der Ausnehmung 10 befindet. Analog kann sich natürlich auf der gegenüberliegenden Seite des zweiten Gelenkarms 4 auch eine untere Wandung der Ausnehmung 10, die ebenfalls zum ersten Gelenkarm 2 gehört, auf der dem zweiten Gelenkarm 4 gegenüberliegenden Seite des Abstandselementes 14 befinden.

Man erkennt in Figur 3, dass das Verbindungselement 12 mit einem Form-schlusselement 26 ausgestattet ist, durch das es mit einem Werkzeug bearbeitbar und handhabbar ist.

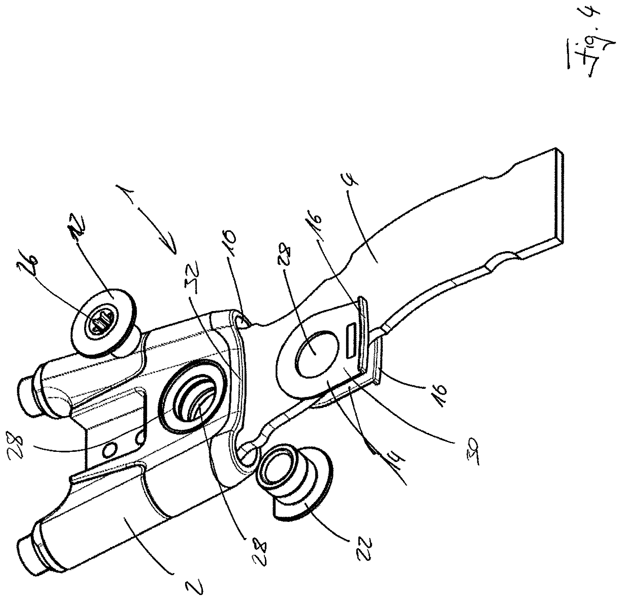

Figur 4 zeigt das Gelenk 1 in teilweise demontierten Zustand. Der erste Gelenkarm 2 verfügt über die Ausnehmung 10, in die der zweite Gelenkarm 4 eingeschoben ist. Die beiden Abstandselemente 14 jedoch sind noch nicht in die Ausnehmung 10 eingeführt. Man erkennt, dass sie über eine Bohrung 28 verfügen, durch die das Verbindungselement 12 und im gezeigten Ausführungsbeispiel auch das Verbindungsgegenelement 22 hindurchgeführt werden können. Das Verbindungsgegenelement 22 verfügt über einen Innengewinde, das zu einem Außengewinde des Verbindungselementes 12 korrespondierend ausgestaltet ist. Im Verbindungselement 12 ist wieder das Formschlusselement 26 dargestellt.

Die beiden Abstandselemente 14 haben wieder die Anschläge 16, die im gezeigten Ausführungsbeispiel zur Hauptfläche 30 des jeweiligen Abstandselementes 14 einen Winkel einschließen, der vorliegend 90° ist. Der Anschlag 16 ist so ausgebildet, dass er an einem Rand 32 der Ausnehmung 10 anliegt, wenn das Ab-standselement 14 wie in den Figuren 1, 2 und 3 dargestellt, in die Ausnehmung 10 eingeschoben ist und das Verbindungselement 12 sowie das Verbindungsgegenelement 22 durch die Bohrung 28 hindurchgeführt ist. Selbstverständlich ist anstelle eines Verbindungsgegenelementes beispielsweise auch eine Mutter für eine Schraube oder ein ähnliches Element denkbar.

Man erkennt in Figur 4, dass der erste Gelenkarm 2 und der zweite Gelenkarm 4 jeweils eine Bohrung 28 aufweisen, die zur Montage des Gelenkes 1 in Überdeckung gebracht werden müssen. Erst dann kann durch die Bohrungen 28 im ersten Gelenkarm 2, im zweiten Gelenkarm 4 sowie in allen vorhandenen Abstands-elementen 14 das Verbindungselement 12 geführt werden.

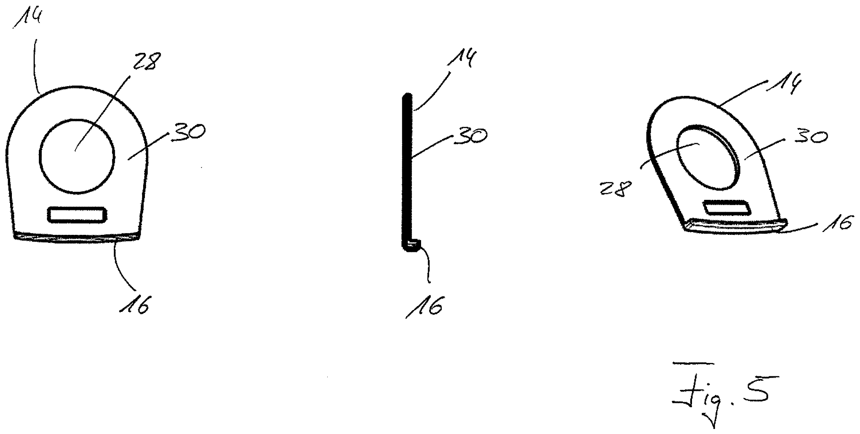

Figur 5 zeigt das Abstandselement 14 in drei unterschiedlichen Ansichten. Man erkennt jeweils, dass das Abstandselement 14 eine Hauptfläche 30 aufweist, in der die Bohrung 28 angeordnet ist. Zudem verfügt das Abstandselement 14 über den Anschlag 16, der leicht gewölbt ausgebildet ist, um den Rand 32 der Ausnehmung 10 zu folgen. Selbstverständlich sind hier auch andere Geometrien möglich.

### Bezugszeichenliste

- 1: Gelenk
- 2: erster Gelenkarm
- 4: zweiter Gelenkarm
- 6: Schwenkachse
- 8: Aufnahmeeinrichtung
- 10: Ausnehmung
- 12: Verbindungselement
- 14: Abstandselement
- 16: Anschlag
- 18: Aufnahmehülse
- 20: Einstelleinrichtung
- 22: Verbindungsgegenelement
- 24: obere Wandung
- 26: Formschlusselement
- 28: Bohrung
- 30: Hauptfläche
- 32: Rand

## Patentansprüche

1. Gelenk (1) für eine orthopädietechnische Einrichtung, das
einen ersten Gelenkarm (2) mit einer Ausnehmung (10),
einen zweiten Gelenkarm (4), der
in die Ausnehmung (10) hineinragt und
darin mit dem ersten Gelenkarm (2) um eine Schwenkachse (6) schwenkbar verbunden ist, und
wenigstens ein Abstandselement (14) aufweist, das
innerhalb der Ausnehmung (10) zwischen dem ersten Gelenkarm (2) und dem zweiten Gelenkarm (4) angeordnet ist und
durch das die Schwenkachse (6) verläuft,
wobei das wenigstens eine Abstandselement (14) von außen zugänglich ist, insbesondere aus der Ausnehmung (10) herausragt, **dadurch gekennzeichnet, dass** wenigstens eines der Abstandselemente (14) einen Anschlag (16) aufweist, der an einem Rand (32) der Ausnehmung (10) anliegt und der verhindert, dass das Abstandselement (14) vollständig in der Ausnehmung (10) zwischen dem ersten Gelenkarm (2) und dem zweiten Gelenkarm (4) aufgenommen werden kann.

2. Gelenk (1) nach Anspruch 1, **dadurch gekennzeichnet, dass** das Gelenk (1) wenigstens zwei Abstandselemente (14) aufweist, die auf unterschiedlichen Seiten des zweiten Gelenkarmes (4) in der Ausnehmung (10) angeordnet sind.

3. Gelenk (1) nach einem der vorstehenden Ansprüche, **dadurch gekennzeichnet, dass** das Abstandselement (14) verdrehsicher zu dem ersten Gelenkarm (2) gelagert ist.

4. Gelenk (1) nach einem der vorstehenden Ansprüche, **dadurch gekennzeichnet, dass** wenigstens eines der Abstandselemente (14) eine Bohrung (28) aufweist, durch die ein Verbindungselement (12) verläuft, durch das der erste Gelenkarm (2) und der zweite Gelenkarm (4) verbunden sind.

5. Gelenk (1) nach einem der vorstehenden Ansprüche, **dadurch gekennzeichnet, dass** der Anschlag (16) so ausgebildet ist, dass er einer Kontur des Randes (32) folgt und insbesondere formschlüssig an der Kontur anliegt.

6. Gelenk (1) nach einem der vorstehenden Ansprüche, **dadurch gekennzeichnet, dass** wenigstens ein Abstandselement (14) aus einem Kunststoff besteht.

7. Orthopädietechnische Einrichtung mit wenigstens einem Gelenk (1) nach einem der vorstehenden Ansprüche.

8. Orthopädietechnische Einrichtung nach Anspruch 7, **dadurch gekennzeichnet, dass** es sich um ein Schienensystem für eine Orthese oder eine Knöchelorthese handelt.

## Claims

1. Joint (1) for an orthopedic device, comprising
a first articulated arm (2) having a recess (10),
a second articulated arm (4), which
protrudes into the recess (10) and
is connected therein to the first articulated arm (2) so as to be pivotable about a pivot axis (6), and
at least one spacer element (14), which
is arranged between the first articulated arm (2) and the second articulated arm (4) within the recess (10) and
through which the pivot axis (6) extends,
wherein the at least one spacer element (14) is accessible from the outside, in particular protrudes out of the recess (10), **characterized in that** at least one of the spacer elements (14) has a stop (16) that abuts an edge (32) of the recess (10) and that prevents the spacer element (14) from being able to be fully received between the first articulated arm (2) and the second articulated arm (4) in the recess (10),.

2. Joint (1) according to claim 1, **characterized in that** the joint (1) has at least two spacer elements (14) arranged in the recess (10) on different sides of the second articulated arm (4).

3. Joint (1) according to any of the preceding claims, **characterized in that** the spacer element (14) is non-rotatably mounted relative to the first articulated arm (2).

4. Joint (1) according to any of the preceding claims, **characterized in that** at least one of the spacer elements (14) has a hole (28) through which a connecting element (12) extends, by means of which the first articulated arm (2) and the second articulated arm (4) are connected.

5. Joint (1) according to any of the preceding claims, **characterized in that** the stop (16) is formed such as to follow a contour of the edge (32) and in particular such as to abut the contour in a positive manner.

6. Joint (1) according to any of the preceding claims, **characterized in that** at least one spacer element (14) consists of a plastics material.

7. Orthopedic device comprising at least one joint (1) according to any of the preceding claims.

8. Orthopedic device according to claim 7, **characterized in that** the device is a splint system for an orthosis or an ankle brace.

## Revendications

1. Articulation (1) pour un dispositif orthopédique, comportant
un premier bras d'articulation (2) ayant un évidement (10),
un second bras d'articulation (4) qui pénètre dans l'évidement (10) et qui est relié, dans ledit évidement, au premier bras d'articulation (2) de façon mobile en basculement autour d'un axe de basculement (6), et
au moins un élément écarteur (14) qui est disposé à l'intérieur de l'évidement (10) entre le premier bras d'articulation (2) et le second bras d'articulation (4) et qui passe par l'axe de basculement (6),
ledit au moins un élément écarteur (14) étant accessible de l'extérieur en particulier en dépassant hors de l'évidement (10),
**caractérisée en ce que**
l'un au moins des éléments écarteurs (14) comprend une butée (16) qui prend appui contre un bord (32) de l'évidement (10) et qui empêche que l'élément écarteur (14) puisse être reçu complètement dans l'évidement (10) entre le premier bras d'articulation (2) et le second bras d'articulation (4).

2. Articulation (1) selon la revendication 1,
**caractérisée en ce que**
l'articulation (1) comprend au moins deux éléments écarteurs (14) qui sont disposés sur différents côtés du second bras d'articulation (4) dans l'évidement (10).

3. Articulation (1) selon l'une des revendications précédentes,
**caractérisée en ce que**
l'élément écarteur (14) est monté de façon bloquée en rotation par rapport au premier bras d'articulation (2).

4. Articulation (1) selon l'une des revendications précédentes,
**caractérisée en ce que**
l'un au moins des éléments écarteurs (14) présente un perçage (28) qui est traversé par un élément de liaison (12) reliant le premier bras d'articulation (2) et le second bras d'articulation (4).

5. Articulation (1) selon l'une des revendications précédentes,
**caractérisée en ce que**
la butée (16) est réalisée de manière à suivre un contour du bord (32) et à prendre appui contre le contour en particulier par coopération de forme.

6. Articulation (1) selon l'une des revendications précédentes,
**caractérisée en ce que**
au moins un élément écarteur (14) est constitué en matière plastique.

7. Dispositif orthopédique comportant au moins une articulation (1) selon l'une des revendications précédentes.

8. Dispositif orthopédique selon la revendication 7,
**caractérisé en ce que**
ledit dispositif est un système d'attelle pour une orthèse ou une orthèse de cheville.
